# EUROPEAN PATENT SPECIFICATION

(11) **EP 1 909 097 B1**
(45) Date of publication and mention of the grant of the patent: **18.04.2012**
(21) Application number: 06757074.7
(22) Date of filing: 06.06.2006
(51) Int. Cl.: G01N 27/327, G01N 27/28, C12Q 1/00

(54) **BIOSENSOR AND BIOSENSOR CELL**
BIOSENSOR UND BIOSENSORZELLE
BIOCAPTEUR ET CELLULE À BIOCAPTEUR

(30) Priority: 06.06.2005 JP 2005165275
(43) Date of publication of application: 09.04.2008
(73) Proprietor: Nikkiso Company Limited, Tokyo 150-0013 (JP)
(72) Inventor: AKAHORI, Yukihiro, c/o NIKKISO Co., Ltd., Makinohara-shi, Shizuoka 4210496 (JP); YAMAZAKI, Hiromi, c/o NIKKISO Co., Ltd., Makinohara-shi, Shizuoka 4210496 (JP)
(74) Representative: GROSSE SCHUMACHER KNAUER VON HIRSCHHAUSEN
(86) International application number: PCT/JP2006/311335
(87) International publication number: WO 2006/132250

(56) References cited:
- JP-A- 5 256 812
- JP-A- 05 256 812
- JP-A- 2001 066 278
- US-A1- 20040 026 244

## Description

### FIELD OF THE INVENTION

The present invention relates to a biosensor and a biosensor cell. Described in more detail, the present invention relates to a biosensor which forms a small biosensor cell with good temperature stability. The present invention also relates to a small biosensor cell which has good temperature stability.

### BACKGROUND OF THE INVENTION

A biosensor is a sensor which uses a biological material or a biologically derived material as a molecular recognition element. For example, enzyme sensors and the like which use enzymes as the biological or biologically derived materials are known. The biosensor is used to measure the concentration of a biological or biologically derived material which has affinity with the biological or biologically derived material that is used as the molecular recognition element. For example, a biosensor measures the concentration of a substrate or coenzyme or the like in blood or in the body. A biological or biologically derived material that has affinity with the biological material that is to be measured is selected as the molecular recognition element used in the biosensor. For example, for a biosensor which measures the glucose concentration in the body or blood (this is also called the sample solution), the enzyme which is selected as the molecular recognition element is glucose oxidase.

The biosensor is also used, for example, in a continuous measurement device which continuously measures the glucose concentration and the like inside the blood vessels or subcutaneous tissue of diabetic patients and the like. The biosensors used in these continuous measurement devices are preferably small, highly sensitive, highly stable, with a long service life.

An example of a conventional biosensor is a biosensor having, on the outside of an electrode having a working electrode and reference electrode or on the outside of a working electrode, an enzyme film layer containing a non-cross-linked hydrophilic polymer and an enzyme dispersed into the non-cross-linked hydrophilic polymer and cross-linked (see patent reference 1). This biosensor is constructed in a tubular shape and is small with excellent sensitivity and stability and has a long service life. However, an even smaller biosensor is preferred.

An example of a conventional biosensor which is capable of miniaturization more than this tubular type of biosensor includes, for example, a plate biosensor (see reference 2) having (1) an electrode system, having at least a measurement electrode and a counter electrode layer provided on top of an insulating substrate; (2) a spacer which is superposed on top of the electrode to form a space opposite a portion of the measurement electrode and counter electrode; (3) a reaction reagent part which is formed in the space, (4) a cover plate which is superposed on the spacer. The space which is surrounded by the substrate and spacer and cover plate forms a capillary for the sample solution pathway. The reaction reagent part contains oxidation reduction enzyme, electron carrier, hydrophilic polymer, and surface active agent. This biosensor is capable of miniaturization. However, for each measurement, the reaction reagent part is dissolved for the oxidation reduction reaction to occur. As a result, in order to use in a continuous measurement device, there is need for increased service life.

A further example of a biosensor is described in JP-5256812. This biosensor constitutes an insulating substrate formed by sintering alumina and a working electrode, reference electrode and counter electrodemade of platinum and a self-regulating temperature control heater all of which are formed on the upper surface of the substrate.

There is an optimal temperature for the enzyme reaction. The activity of the enzyme changes depending on the temperature. Therefore, when the measurement temperature changes, the measurement values also fluctuate, and as a result, the measurement accuracy of the biosensor is reduced. For example, with a biosensor which uses glucose oxidase, when the measurement temperature fluctuates 1 degree C, the measurement concentration fluctuates approximately 5 mg/dL.

Therefore, when using a biosensor to measure the concentration of a biological or biologically derived material in a sample solution, the measurement temperature must be maintained constant. Methods for this include, for example, placing the biosensor in an incubator, and for each time it is used, the temperature of the incubator is adjusted. In addition, another method is one in which the enzyme activity of the enzyme in the enzyme reaction is adjusted according to the measurement temperature.
Patent reference 1: Japanese Laid-Open Patent Publication Number Heisei 8-5601
Patent reference 2: Japanese Laid-Open Patent Publication Number 2000-221157

However, with regard to the method for adjusting the temperature of an incubator, the temperature of the entire incubator in which the biosensor is placed must be maintained. As a result, in order for the temperature of the biosensor (measurement temperature) to reach a constant temperature, for example, 30 minutes or greater is needed, which results in the elongation of the preparation time before_the initiation of measurement. In addition, the device for maintaining the temperature of the incubator is large and complex.

On the other hand, the method for modifying the activity of the enzyme is not feasible, because the activities of enzymes at a temperature differ from each other, and if the temperature changes moment by moment during a measurement, it may be difficult to control the activity sufficiently. For these reasons, a biosensor which solves these problems is desired.

### DISCLOSURE OF THE INVENTION

### PROBLEMS TO BE SOLVED

The object of the present invention is to provide a biosensor which is used for a miniature biosensor cell which has a high level of temperature stability. In addition, the object of the present invention is to provide a miniature biosensor cell having a high degree of temperature stability.

### MEANS FOR SOLVING PROBLEMS

In order to solve the problems, claim 1 is directed to a biosensor that includes a supporting layer, on a front surface of which a reference electrode layer, a working electrode layer, a counter electrode layer, and a temperature detection means are formed, an enzyme film that coats a surface of the working electrode layer, and a heater member formed on a back surface of the supporting layer. Claim 2 is directed to the biosensor according to claim 1, in which the supporting layer is a film. Claim 3 is directed to the biosensor according to claim 1, in which the heater member is formed on a surface of a base substrate, and the supporting layer, which is formed on a surface of the heater member, is a resist insulating layer. Claim 4 is directed to a biosensor cell that includes a sample chamber to which a sample solution flows in and out, and a biosensor according to any one of claims 1-3, in which the reference electrode layer and the counter electrode layer are positioned at the sample chamber so as to be capable of making contact with the sample solution, the temperature detection means is positioned at the sample chamber so as to be capable of measuring temperature of the sample solution, and the enzyme film, which coats the working electrode layer, is positioned at the sample chamber so as to be capable of making contact with a substance to be measured in the sample solution.

### EFFECT OF THE INVENTION

According to the biosensor of the present invention, the temperature detection means and the heater member are on top of the supporting layer which is formed as a plate which is capable of miniaturization. As a result, the temperature of the sample solution and the working electrode layer and other components of the biosensor (referred to as the sample solution and the components) is detected by the temperature detection means. Based on this temperature detection, temperature adjustment of the sample solution and the components is possible with the heater member. As a result, miniaturization of the biosensor is possible, and in addition, the temperature is always maintained constant.

According to the biosensor cell of the present invention, because it has the biosensor of the present invention, there is no need to use a complex, large temperature adjusting device such as an incubator or the like. Therefore, the biosensor cell of the present invention effectively takes advantages of the small size of the biosensor, and as a result, the structure is simple and miniaturization is possible. In addition, there are fewer occurrences of failure and the like.

In addition, according to the biosensor cell of the present invention, because it has a sample chamber and the biosensor of the present invention, the temperature adjustment of the sample solution and the components in the sample chamber is easy. As a result, the temperature of the sample solution and the components is adjusted to the desired temperature in a short period of time. In addition, there is rapid response to any temperature changes in the sample solution and the components, and the temperature of the sample solution and the components is maintained constant. Therefore, the biosensor cell of the present invention has a high degree of temperature stability and measures the biological material with great accuracy.

### BRIEF DESCRIPTION OF THE DRAWINGS

Fig. 1 is a schematic perspective view showing one embodiment of a biosensor of the present invention. Fig. 1(a) is a schematic perspective view showing a front surface of the biosensor. Fig. 1(b) is a schematic perspective view showing its back surface.
Fig. 2 is a schematic top view of a biosensor chip which is capable of forming one embodiment of the biosensor of the present invention.
Fig. 3 is a schematic top view of a biosensor chip which is capable of forming one embodiment of the biosensor of the present invention. Fig. 3(a) is a schematic top view showing one of the surfaces of a supporting layer. Fig. 3(b) is a schematic top view showing another one of the surfaces of the supporting layer.
Fig. 4 is a schematic top view showing the front surface of the biosensor of another embodiment of the present invention.
Fig. 5 is a schematic cross-sectional view showing one embodiment of a biosensor cell of the present invention.
Fig. 6 is a schematic top view showing one embodiment of the biosensor cell of the present invention.
Fig. 7 is a schematic exploded perspective view showing one embodiment of the biosensor cell of present invention.
Fig. 8 is a schematic partial cross-sectional view showing a usage example of the biosensor.
Fig. 9 is a schematic cross-sectional view of a supporting layer provided with an electrode and a heater member, according to one embodiment of the biosensor.
Fig. 10 is a schematic view of one embodiment of the biosensor cell.
Fig. 11 is a schematic view of another embodiment of the biosensor cell.

### DESCRIPTION OF THE REFERENCE NUMERALS

- 1,2: biosensor
- 5, 6: biosensor chip
- 10: supporting layer
- 10a, 10b, 10c, 10d 10e, 10f: surface
- 11: reference electrode layer
- 12: working electrode layer
- 13: counter electrode layer
- 14: temperature detection means (thermistor)
- 11a, 12a, 13a, 14a, 14b, 15a, 15b: wires
- 11b, 12b, 13b: ground layer
- 15: heater member
- 16: enzyme film
- 17: protective film
- 20: biosensor cell
- 21: sample chamber
- 22a, 22b: transport pipe
- 23: upper cover member
- 24: gasket
- 24a: opening
- 25: lower cover member
- 25a: insertion part
- 26a, 26b: fastener
- 26c: slit
- 30: measurement part
- 31: temperature control part
- 30a, 31a, 31b: interconnection
- 32: transport means
- A: fold line
- 40: substrate
- 41: heater member
- 42: supporting layer
- 43: electrode layer
- 50: biosensor
- 51: biosensor cell
- 52: lower member

### BEST MODE OF THE INVENTION

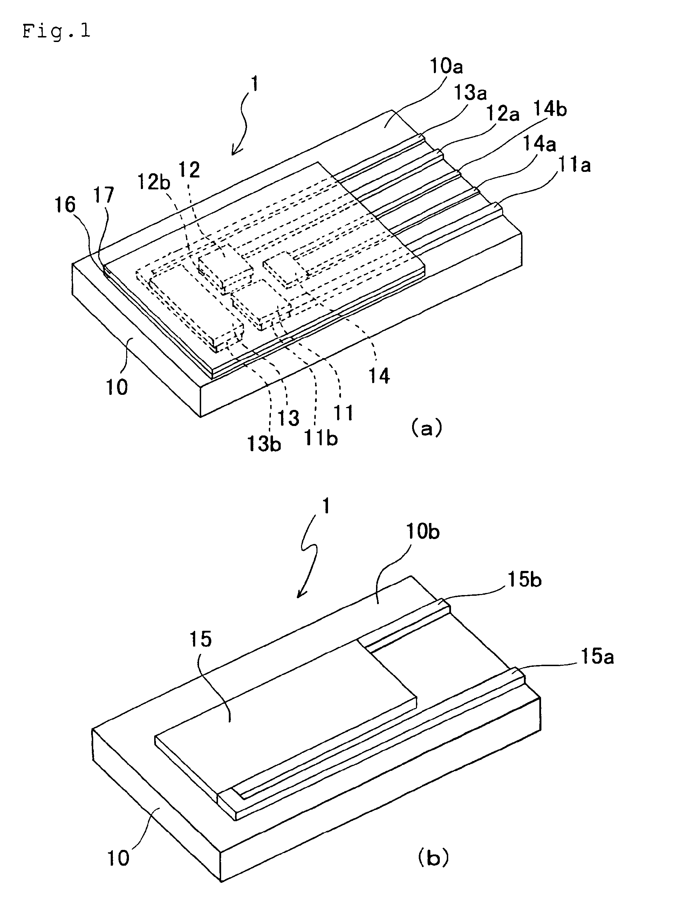

As shown in Figs. 1(a) and 1(b), a biosensor 1, which is one example of the present invention, has: a supporting layer 10; a reference electrode layer 11, a working electrode layer 12, a counter electrode 13, and a temperature detection means 14 on a front surface 10a of the supporting layer 10; an enzyme layer 16 which is formed on a surface of the working electrode layer 12; and a heater member 15 which is formed on a back surface 10b of the supporting layer 10. In this biosensor 1, on the surface 10a of the supporting layer 10, there are the reference electrode layer 11, the working electrode layer 12, the counter electrode layer 13 and the temperature detection means 14, and on their surfaces, the enzyme layer 16 is formed. On the other surface 10b of the supporting layer 10, the heater member 15 is formed.

According to an embodiment shown in Fig. 1, the supporting layer 10 is formed as a plate. Thus, miniaturization of the biosensor is possible. As long as miniaturization is possible, the size or thickness of the supporting layer 10 is not limited. The material for forming the supporting layer 10 is an insulating material. Examples include plastics such as polyethylene, polyethylene terephthalate, and the like, ceramics, glass, paper, and the like. The supporting layer 10 for the biosensor 1 is formed from polyethylene. A thickness of that supporting layer 10, which is formed from polyethylene, is usually 100-250 µm.

As shown in Fig. 1(a), the rectangular counter electrode 13 is positioned near one end of the front surface 10a of the supporting layer 10. The size and shape and the like of the counter electrode 13 is not limited. However, the thickness of the counter electrode 13 should be adjusted to one between 5-100 µm. The material for forming the counter electrode 13 is a conductive material which does not deteriorate even when in contact with the sample solution. The material for the counter electrode 13 also has to have a stable electric potential. Examples include metals such as aluminum, nickel, copper, platinum, gold, and silver and the like, conductive metal oxides such as ITO and the like, carbon materials such as carbon and carbon nanotube and the like. Among these materials, carbon materials are preferred because the counter electrode 13 is easily formed by screen printing of a paste of carbon material. The counter electrode 13 of the biosensor I is formed from carbon.

On the front surface 10a of the supporting layer 10, the rectangular working electrode layer 12 is positioned next to the counter electrode layer 13. The size and shape of the working electrode layer 12 is not limited, however, the thickness should be adjusted to between 5 and 100 µm. The material for forming the working electrode layer 12 is a conductive material which does not deteriorate even when in contact with the sample solution. The material for the working electrode layer 12 also has to have a stable electric potential. The working electrode layer 12 uses the same conductive materials as the counter electrode 13. The working electrode layer 12 of the biosensor 1 is formed from carbon.

On the front surface 10a of the supporting layer 10, the rectangular reference electrode layer 11 is positioned adjacent to the working electrode layer 12 and the counter electrode layer 13. The size and shape and the like of reference electrode layer 11 is not limited, however the thickness should be adjusted to between 5 and 100 µm for example. The material for forming the reference electrode layer 11 is a conductive material which does not deteriorate even when in contact with the sample solution. The material for the reference electrode layer 11 also has to have a stable electric potential. Examples of materials for the reference electrode layer 11 include the metals, the conductive metal oxides, the carbon materials, and the materials that are combination of the previously described metals and their salts. Among these materials, silver/silver chloride is preferred because it is not readily ionized at the electrode layer surface. The reference electrode layer 11 of the biosensor 1 is formed from silver/silver chloride.

Between the supporting layer 10 and the reference electrode layer 11, the working electrode layer 12 and the counter electrode layer 13, there are ground layers 11b, 12b, and 13b. The ground layers 11b, 12b, and 13b are similar in shape as the reference electrode layer 11, the working electrode layer 12, and the counter electrode layer 13, but they are slightly smaller in size. The material which forms the ground layers 11b, 12b, and 13b are conductive materials, for example metals, conductive metal oxides, carbon materials, and the like. Among these materials, low resistance materials such as silver, platinum and the like are preferred.

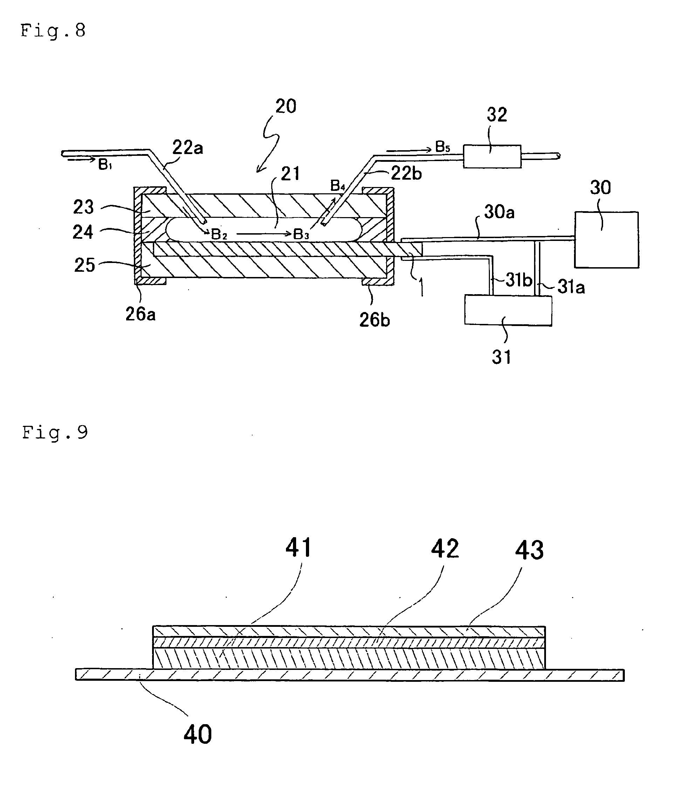

The reference electrode layer 11, the working electrode layer 12, and the counter electrode layer 13 are provided with wires 11a, 12a, and 13a. The wires 11a, 12a, and 13a reach to the other end of supporting layer 10. Through the wires 11a, 12a, and 13a, each of the electric potentials for the reference electrode layer 11, the working electrode layer 12, and the counter electrode layer 13 goes to a measuring part 30 (see Fig. 8). The wires 11a, 12a, and 13a should be formed with the same material and same thickness as the ground layers 11b, 12b, and 13b.

On the front surface 10a of the supporting layer 10, the rectangular temperature detection means 14 is positioned next to the reference electrode layer 11 and the working electrode layer 12. The temperature detection means 14 is for detecting the temperature of the sample solution and the components. For example, a thermocouple, resistance thermometer, thermistor, or the like may be selected. The size and thickness is not limited. Examples of the shapes of the temperature detection means 14 include bead, disk, rod, thin film, chip and the like. In the biosensor 1, a thermistor, which is molded into a thin film form, is selected as the temperature detection means 14. In general, a thermistor can be made very small in size. For example, if a thermistor is a thin film type, its thickness can be made as small as 0.15-0.25 mm. Therefore, the selection of the thermistor contributes to making the biosensor thinner. Also, in practical use, the thermistor is capable of detecting minute temperature of about 2/10,000, temperature management of the biosensor is made very accurate. The material used to form the thermistor can be any semiconductor material that is, for example, a metal oxide of iron, nickel, manganese, cobalt, titanium or the like.

The temperature detection means 14 is provided with wires 14a and 14b which reach to the other end of the supporting layer 10. Through the wires 14a and 14b, the temperature of the sample solution detected by the temperature detection means 14 is transmitted to a temperature control part (see Fig. 8). The material which forms the wires 14a and 14b is a conductive material. Examples include, previously described metals, conductive metal oxides, carbon materials, and the like. Among these materials, low resistance materials such as silver and the like is preferred.

In the biosensor shown in Fig. 1(a), the enzyme film 16 is formed on the surface of the reference electrode layer 11, the working electrode layer 12, the counter electrode 13, and the temperature detection means 14. The enzyme film 16 is a film with an enzyme or an enzyme and mediator which are immobilized. In this regard, it is enough if the enzyme film coats the surface of the working electrode layer 12, and not necessary that the enzyme film coats the surfaces of the reference electrode layer 11, the counter electrode layer 13 and the temperature detection means 14. It is preferable that the temperature detection means 14 is provided so as to be exposed to the sample solution, or to be directly in contact with the sample solution, without being coated by the enzyme film, so that temperature detection can be performed accurately. Here, in the biosensor shown in Fig. 1(a), the surfaces of the reference electrode layer 11, the counter electrode layer 13, and the temperature detection means 14 are covered by the enzyme film, so that a protective film, which is described below, can be conveniently stuck onto it. In this regard, although the enzyme film coats the surfaces of the reference electrode layer and the counter electrode layer, the sample solution is practically capable of making contact with the reference electrode layer and the counter electrode layer because the sample solution permeates or diffuses into the enzyme film.

The immobilized enzyme is selected based on the biological materials that will be measured. For example, if the biological or biologically derived material to be measured is alcohol, alcohol oxidase may be selected; if it is glucose, β-D-glucose oxidase may be selected; if it is cholesterol, cholesterol oxidase may be selected; if it is phosphatidylcholine, phospholipase and choline oxidase may be selected; if it is urea, urease may be selected; if it is uric acid, uricase may be selected; if it is lactic acid, lactate dehydrogenase may be selected; if it is oxalic acid, oxalic decarboxylase may be selected; if it is pyruvic acid, pyruvate oxidase may be selected; if it is ascorbic acid, ascorbate oxidase may be selected; and if it is trimethyl amine, a flavin containing mono oxidase may be selected. The biosensor chip of this example is constructed as a glucose sensor with β-D-glucose oxidase selected as the enzyme.

Examples of the mediators which are to be immobilized include conductive materials capable of oxidation-reduction such as ferrocene derivatives, 1,4-benzoquinone, tetrathiafulvalene, ferricinium ion, hexacyano iron (III) ion, potassium hexacyano ferrate, methylene blue and the like.

The method for immobilizing the enzyme and/or the mediator (henceforth referred to as enzyme composition) includes, for example, carrier binding method, cross-linking method and entrapment method and the like. In the carrier binding method, a water-insoluble carrier is bound to an enzyme composition and immobilized. Examples of carrier binding include covalent binding method, ion binding method, physical adsorption method and the like. In the cross-linking method, the enzyme composition is reacted with a reagent (cross-linking agent) which has two or more functional groups, and the enzyme or the enzyme composition immobilized by cross-linkages. In the entrapment method, the enzyme composition is entrapped inside a fine matrix such as a gel and the like. The enzyme composition is covered by a semi-permeable polymer film.

The carrier used in the carrier binding method is not limited as long as it is a water-insoluble polymer material. Examples include derivatives of polysaccharides, such as methyl cellulose, ethyl cellulose, hydroxyethyl cellulose, carboxy methyl cellulose, cellulose acetate, and the like; porous polyurethane; polyvinyl alcohol; metal alginate; sodium polyacrylate; polyethylene oxide; and the like. The cross-linking agent used in the cross-linking method is a reagent with two or more functional groups. Examples include glutaraldehyde, isocyanate derivative, bisdiazobenzene and the like. For the polymer compound used in the entrapment method, examples include polyacrylamide, polyvinyl alcohol and the like.

The amount of enzyme that is immobilized is set according to the type of enzyme and usage. In addition, with the carrier binding method and entrapment method, the amount of enzyme contained in the carrier and in the gel and the like is also set as appropriate. For example, the amount of enzyme with respect to the total weight of the enzyme film that is formed is around 0.02-4% by mass, and preferably 0.02-0.2% by mass.

In the biosensor 1, the enzyme film 16 is formed by the entrapment method in which the enzyme composition is immobilized inside a polyvinyl alcohol by mixing the enzyme composition with a polyvinyl alcohol such as PVA-SbQ (for example, made by Toyo Gousei Kogyo).

In PVA-SbQ, the base polymer is a completely saponified or partially saponified polyvinyl alcohol, and a stilbazolium group is added as a photosensitive pendant group. This is a polyvinyl alcohol in which several mol %, for example 1-5 mol %, of the hydroxyl groups of the base polymer is substituted with the photosensitive group. Examples of the photosensitive group include styryl pyridinium group, styryl quinolinium group and the like. Examples of the polyvinyl alcohol type photosensitive polymer include product number SPP-H-13 (degree of polymerization of the polyvinyl alcohol is 1700, saponification rate is 88%, SbQ content is 1.3 mol %), product number SPP-M-20 (degree of polymerization of the polyvinyl alcohol is 1200, saponification rate is 88%, SbQ content is 2.0 mol %), product number SPP-L-30 (degree of polymerization of the polyvinyl alcohol is 600, saponification rate is 70%, SbQ content is 3.0 mol%), product number SPP-S-10 (degree of polymerization of the polyvinyl alcohol is 2300, saponification rate is 88%, and SbQ content is 1.0 mol%), and the like.

Regarding the method of immobilizing the enzyme composition in this polyvinyl alcohol type photosensitive polymer compound, first, the enzyme composition is dissolved or dispersed uniformly in an aqueous solution of PVA-SbQ. This solution or dispersion solution is cast on top of a smooth and transparent plate and dried. Next, using a light source which emits a light of wavelength 300-370 nm (for example, sunlight, fluorescent lamp, chemical lamp, xenon lamp, and the like), the film is exposed to light from both sides. The SbQ groups are cross-linked through a dimerization reaction by the light. As a result, the enzyme composition is immobilized in the polyvinyl alcohol type photosensitive polymer compound, and an immobilized enzyme film is achieved.

In the biosensor 1, a protective film 17 is formed on the surface of the enzyme film 16. The protective film 17 protects the working electrode layer 12 and the like. In addition, the protective film 17 is a film which allows the biological materials or the like that are to be measured to permeate through to the working electrode layer 12. The protective film 17 is a film with these functions. For example, the protective film 17 is a film formed from a polymer material that is water insoluble. Other examples of the protective film 17 include films and the like formed from polymer material with pores of the desired diameter, such as polycarbonate, polyvinyl alcohol, cellulose acetate, polyurethane and the like. In order to form pores in the film formed from the polymer material, it is preferable to provide a tracking process to track the film formed from the polymer material by heavy ions with heavy energy, and an etching process to form pores by immersing the film being tracked in an etching solution.

As shown in Fig. 1(b), the rectangular heater member 15 is positioned on the back surface 10b of the supporting layer 10. Based on the temperature detected by the temperature detection means 14, the heater member 15 heats the sample solution and the components inside the sample chamber 21 through the supporting layer 10 and maintains the temperature of the sample solution and the components at the desired temperature. For example, when the enzyme is β-D-glucose oxidase, the temperature is maintained at approximately 37 degrees C by the heater member 15.

The size and shape of the heater member 15 is not limited. The thickness is adjusted to between 5 and 100 µm for example. The material for forming the heater member 15 is a material which generates heat with electrification and the like. Examples of the material include the metals described previously, conductive metal oxides, carbon materials, and the like. Among these materials, carbon material is preferred.

The heater member 15 is provided with wires 15a and 15b which reach to the end of the supporting layer 10. Based on the temperature detected by temperature detection means 14, current runs from the temperature control part 30 (see Fig. 8) to the heater member 15, and the heater member 15 generates heat. The material for forming the wires 15a and 15b are a conductive material. Examples include the previously described metals, conductive metal oxides, carbon materials, and the like. Among these materials, low resistance materials such as silver and the like is preferred.

The biosensor 1, which is one example of the present invention, is made as follows. First, the supporting layer 10 is formed into a film with the desired size and thickness through a molding technique such as injection molding, extrusion molding, press molding and the like using the previously described materials.

Next, using the previously described materials, the ground layers 11b, 12b, and 13b are formed in a pattern corresponding to the placement pattern of the reference electrode layer 11, the working electrode layer 12, and the counter electrode layer 13 on the one surface 10a of the supporting layer 10. The ground layers are formed by thin film formation techniques such as vapor deposition, sputtering, plating, etching, printing, and the like. The ground layers are preferably formed by screen printing. In addition, the wires 11a, 12a, 13a, 14a, and 14b extending from the reference electrode layer 11, the working electrode 12, and the counter electrode 13 to the other end of the supporting layer 10 are formed on the surface 10a of the supporting layer 10 using the previously described materials. The methods for forming these wires are the thin film formation techniques described previously. If the wires and the ground layer are to be formed with the same materials, then the wires and the ground layer are preferably formed simultaneously.

On the surface 10a of the supporting layer 10, the reference electrode layer 11, the working electrode layer 12, and the counter electrode layer 13 are each formed by the previously described thin film formation techniques using the materials described above. These electrode layers are preferably formed by the screen printing method. In addition, the temperature detection means 14 is formed on the surface 10a of the supporting layer 10 by the previously described thin film formation techniques using semiconductor materials. The temperature detection means 14 may be formed directly on the supporting layer 10, or the temperature detection means 14 may be formed in advance into a thin film, chip, rod, or the like, and the temperature detection means 14 is then superposed and stuck onto the supporting layer 10 by a conductive adhesive or the like.

The enzyme film 16 is formed so as to cover the reference electrode layer 11, the working electrode 12, the counter electrode 13, and the temperature detection means 14. The enzyme film 16 is formed by dip coating, spray coating, screen printing, dispensing, and the like using the enzyme immobilized by the previously described enzyme immobilization methods.

The protective layer 17 is formed on the surface of the enzyme film 16 from the materials described previously. The protective layer 17 may be formed by dip coating, spray coating, screen printing and the like using a solution of water insoluble polymers. However, the protective film 17 is preferably formed by superposing a thin film in which pores of a desired pore size are formed by electron beam or the like in a film formed in advance from the previously described polymer material.

The heater member 15 is formed on the other surface 10b of the supporting layer 10 as described below. On the surface 10b of the supporting layer 10, the wires 15a and 15b which extend to the end of the supporting layer 10 are formed by the thin film formation technique as previously described using the previously described materials. In addition, on the surface 10b of the supporting layer 10, the heater member 15 is formed by the thin film formation techniques using the materials described previously. As a result, the biosensor 1 having the pattern indicated in Figs. 1 (a) and (b) is formed.

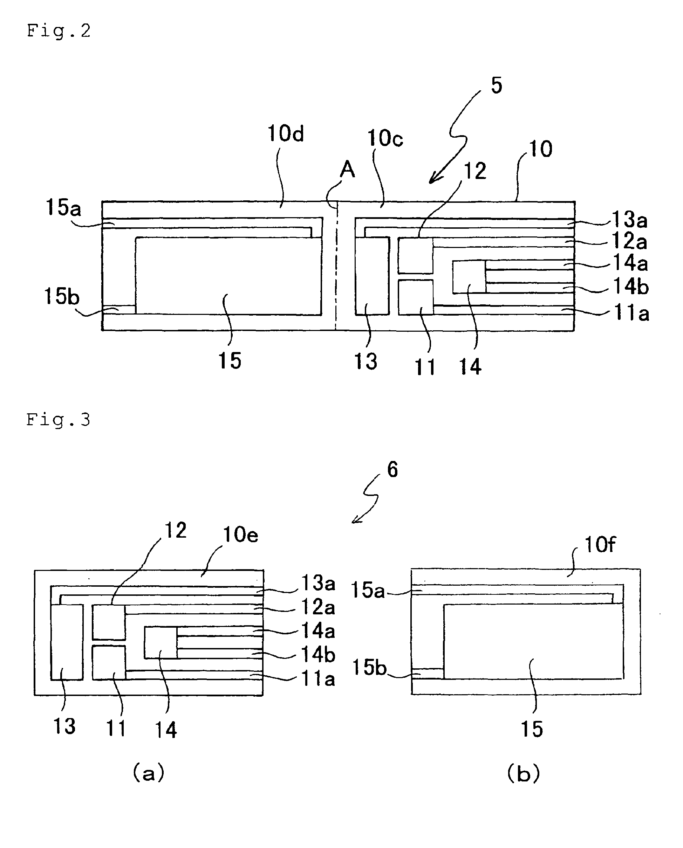

The biosensor 1 shown in Figs. 1(a) and (b) is constructed from a single supporting layer 10. The biosensor of the present invention may also be constructed from a plurality of substrates. For example, a biosensor including biosensor chips 5 and 6 shown in Figs. 2 and 3 is an example of a biosensor constructed with two substrates.

The biosensor chip 5 is shown in Fig. 2. One supporting layer 10 is demarcated by a fold line A formed in approximately the middle of the surface, and a surface 10c is on one side of the line. On the surface 10c, the reference electrode layer 11, the working electrode layer 12, the counter electrode layer 13, the temperature detection means 14, and the enzyme film 16 (not shown) are formed in the same pattern as in the biosensor 1. In addition, on the surface 10d which is on the other side of fold line A, the heater member 15 is formed in the same pattern as in the biosensor 1. In addition, in the biosensor chip 5, the ground layers 11b, 12b, and 13b, the wires 11a, 12a, 13a, 14a, 14b, 15a, and 15b, the protective layer 17 (not shown) are each formed in the same construction as those of the biosensor 1. The biosensor chip 5 is folded at the fold line indicated by the broken line A in Fig. 2. When the two portions of the back surface of the supporting layer 10 are joined to each other, the biosensor is formed. The resulting biosensor has the supporting layer 10, and on the front surface 10c of the supporting layer 10, the reference electrode layer 11, the working electrode layer 12, the counter electrode layer 13, and the temperature detection means 14 are formed, and on their surfaces, the enzyme film 16 is formed, and the heater member 15 is formed on the back surface 10d. of the supporting layer 10.

The biosensor chip 5 is produced basically in the same way as the biosensor I, and one surface of the supporting layer 10 has the same patterns as the front surface 10a and the back surface 10b of the biosensor 1. When the wires 15a and 15b are formed from the same materials as the wires 11a, 12a, 13a, 14a, and 14b, and the ground layer 11b, 12b, and 13b, these are preferably formed simultaneously. The resulting biosensor chip 5 is folded along the broken line A of Fig. 2, and the two portions of the back surface of the supporting layer 10 are joined to each other to make the biosensor chip. The joining of the two portions of the back surface of the supporting layer 10 is through double-sided tape, adhesives or the like, or they can be joined by providing latches and the like. In addition, they may be joined physically by a clip or the like. Accordingly, the biosensor I can be manufactured by a simple operation in which the reference electrode layer 11, the working electrode layer 12, the counter electrode layer 13, the temperature detection means 14, and the wires 11a, 12a, 13a, 14a, 14b, which are formed on the front surface of the biosensor 1, are formed on one side of the supporting layer that can become the supporting layer 10 of the biosensor 1 when folded into two, and the wires 15a and 15b are formed on the other side of the supporting layer that can become the supporting layer 10 of the biosensor 1 when folded into two, all at once by utilizing, for example, a printing method, and, then, the back surface of the supporting layer are folded into two to join the two to each other.

The biosensor chip 6 shown in Fig. 3 is constructed from two substrates 10e and 10f. As shown in Fig. 3(a), the biosensor chip 6 has the supporting layer 10e in which, on one surface, the reference electrode layer 11, the working electrode layer 12, the counter electrode layer 13, and the temperature detection means 14 are formed in the same pattern as the biosensor I, and over these surfaces, the enzyme film (not shown) is formed. As shown in Fig. 3(b), the biosensor chip 6 has the supporting layer 10f in which, on one surface, the heater member 15 is formed in the same pattern as the biosensor 1. In addition, on the substrates 10e and 10f of the biosensor chip 6, the ground layers 11b, 12b, and 13b, the wires 11a, 12a, 13a, 14a, 14b, 15a, and 15b, and the protective layer 17 (not shown) are all formed with the same constructions as those of the biosensor 1. The biosensor is formed by joining together the other surfaces of the substrates 10e and 10f of the biosensor chip 6. The resulting biosensor has the supporting layer 10, and on the front surface 10e of the supporting layer 10, the reference electrode layer 11, the working electrode layer 12, the counter electrode layer 13, and the temperature detection means 14 are formed, and on their surfaces, the enzyme film 16 is formed, and the heater member 15 is formed on the back surface 10f of the supporting layer 10.

The substrates 10e and 10f of the biosensor chip 6 are manufactured in the same basic manner as the biosensor 1 so that the substrates 10e and 10f have the same patterns as the front surface 10a and the back surface 10b of the biosensor 1, respectively. For the resulting substrates 10e and 10f, the other surfaces of each are joined by the joining method described previously in order to form the biosensor chip.

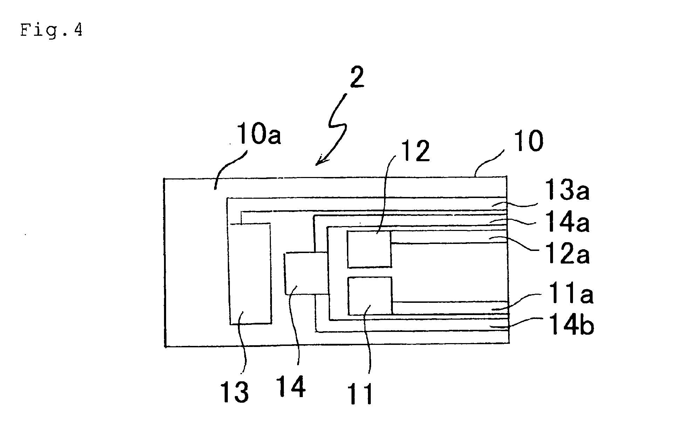

With the biosensor of the present invention, there may be modifications to the patterns of the working electrode and the other components. For example, as shown in Fig. 4, the temperature detection means 14 may be placed between the counter electrode 13 and the alignment of the reference electrode layer 11 and working electrode 12 on the front surface 10a of the supporting layer 10. The biosensor 2 has the same function as the biosensor 1 and is manufactured in the same manner.

The biosensors 1 and 2 have the temperature detection means 14 and the heater member 15 on the supporting layer 10 which is formed as a plate which is capable of miniaturization. The temperature of the sample solution and the components is detected by the temperature detection means 14, and based on this temperature, temperature adjustments of the sample solution and the components are made by the heater member 15. As a result, the biosensors 1 and 2 are capable of miniaturization, and in addition the temperature is maintained constant.

In addition, according to the biosensors 1 and 2, by taking advantage of their size, the construction is simple, and miniaturization is possible. In addition, the biosensors I and 2 have a high degree of heat stability. In this regard, a biosensor which measures biological or biologically derived materials with great precision is formed.

Various modifications, other than the placements shown in Figs. 1 and 4, to the placement of the reference electrode 11, the working electrode layer 12, the counter electrode layer 13, and the temperature detection means 14 are possible for the biosensors I and 2. Furthermore, the enzyme film 16 of the biosensors 1 and 2 is formed to cover the reference electrode layer 11, the working electrode layer 12, and the counter electrode 13. However, having the enzyme film formed on the surface of the working electrode layer 12 is sufficient. It is preferable that the temperature detection means 14 is exposed without being coated by the enzyme film so that temperature detection can be performed accurately.

The biosensors 1 and 2 are used to measure the concentration of biological or biologically derived materials in a sample solution. The format for the biosensors 1 and 2 is not limited. For example, the biosensors may be built into a batch measurement device such as a portable biosensor device and the like. The biosensor may also be built into a continuous measurement device of an artificial pancreas device or the like.

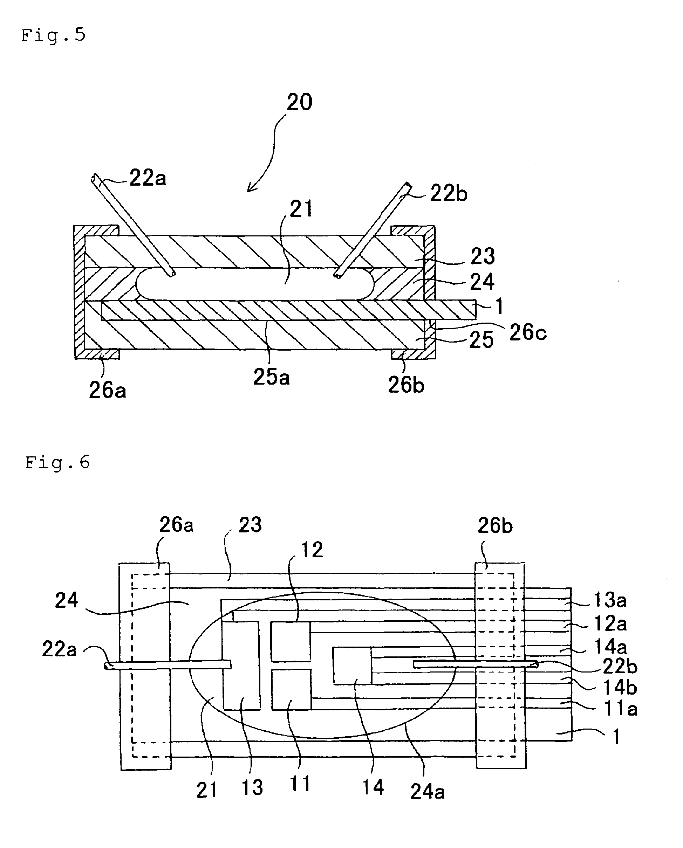

A biosensor cell having the biosensor of the present invention is described below. As shown in Figs. 5 and 6, as an example of the present invention, a biosensor cell 20 is constructed from a sample chamber 21 and the biosensor 1 which is placed at a prescribed position. As shown in Fig. 6, the prescribed position for placing the biosensor I is a position at which the reference electrode layer 11, the counter electrode layer 13, and the temperature detection means 14, and the enzyme film 16 of the biosensor 1 are exposed to the sample chamber 21.

The biosensor cell 20 has a transport pipe 22a and 22b through which the sample solution flows in and out, an upper cover member 23, a gasket 24, a lower cover member 25, fasteners 26a and 26b, and the biosensor 1. The sample chamber 21 in which the sample solution flows in and out is formed from the upper cover member 23 and the gasket 24 and the biosensor 1.

The lower cover member 25 is formed as a plate. An insertion part 25a for the insertion of the biosensor 1 is formed in the lower cover member 25. This insertion part 25a is the same size as the biosensor 1, and the depth of the insertion part 25a is adjusted to a depth which is slightly smaller than the thickness of the biosensor 1. The material for forming the lower cover member 25 is a material which is heat insulating. Examples include plastic, glass and the like. When the lower cover member 25 is formed from a heat insulating material, the biosensor is isolated from the temperature of the surrounding area, and the temperature control of the biosensor cell can be conducted easily. The biosensor cell 20 is formed from polycarbonate.

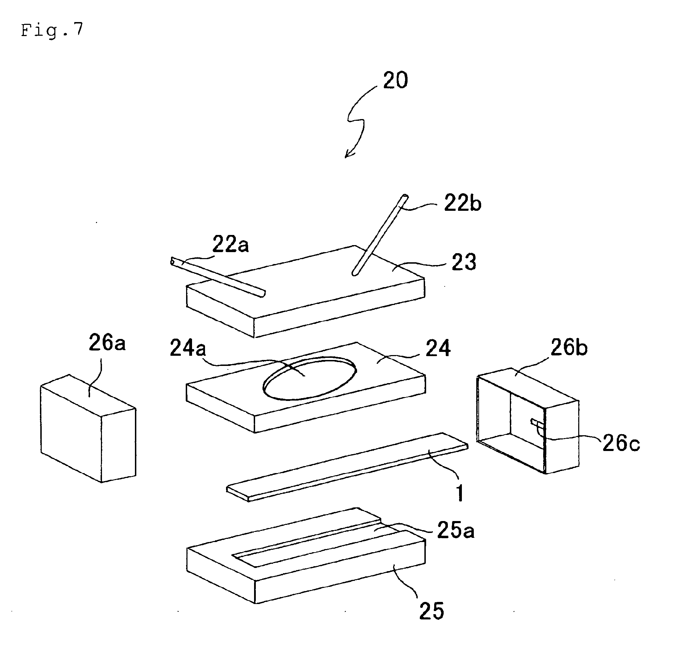

As shown in Fig. 7, the gasket 24 is formed as a sheet unit which has an opening 24a which forms the side wall of the sample chamber 21. The gasket 24 is mounted on top of the lower cover member 25 and the biosensor 1. The gasket 24 forms the side wall of the sample chamber 21 and also seals the sample chamber 21. The material for forming the gasket 24 is not limited. Examples include plastic, rubber and the like. In the biosensor cell 20, the gasket 24 is formed of silicone rubber.

Referring to Fig. 5, the upper cover member 23 is mounted on the gasket 24 and is the ceiling surface for the sample chamber 21. The upper cover member 23 is a plate, and transport pipe holes are formed through the upper cover member 23. The transport pipes 22a and 22b are inserted into the transport pipe holes of the upper cover member 23. The material for forming the upper cover member 23 is not limited, and the upper cover member 23 is formed from the same material as the lower cover member 25.

The transport pipes 22a and 22b are inserted into the transport pipe holes formed in the upper cover member 23. The sample solution which contains the biological material and the like to be measured flows into the sample chamber 21 through the transport pipes 22a and 22b. In addition, the transport pipes 22a and 22b remove the sample solution from the sample chamber 21. The material for forming the transport pipes 22a and 22b are not limited. Examples of materials include plastic, rubber, glass, metal, and the like.

Fasteners 26a and 26b are fastened to the laminate which includes the lower cover member 25, the biosensor 1, the gasket 24, and the upper cover member 23. The fasteners 26a and 26b secures the laminate so that the sample chamber 21 is sealed. As shown in Figs. 5 and 7, in the biosensor cell 20, the fasteners 26a and 26b are formed as caps with a cross-section in the shape of a square bracket. The fasteners 26a and 26b are fastened to the laminate so as to hold the stacked layers together. Therefore, the fastener 26b has a slit 26c through which the biosensor 1 passes. The material for forming the fasteners 26a and 26b is not limited as long as the material has strength to secure the laminate. Examples include plastic, metal, and the like.

The biosensor cell 20 is manufactured as follows. First, the transport pipes 22a and 22b, the upper cover member 23, the gasket 24, the lower cover member 25, and the fasteners 26a and 26b are each formed. The upper cover member 23 and the lower cover member 25 are molded into a plate of the desired size and thickness through molding techniques such as injection molding, extrusion molding, press molding and the like using the previously described materials. The upper cover member 23 has transport pipe holes which are formed through the upper cover member 23. The lower cover member 25 has the insertion part 25a for insertion of the biosensor 1. The gasket 24 is molded into a sheet having the opening 24a by the previously described molding techniques using the previously described materials. The transport pipes 22a and 22b are molded using the previously described materials. For the fasteners 26a and 26b, the previously described materials are molded into caps with a cross section in the shape of a square bracket using previously described molding techniques. The fastener 26b has the slit 26c for the passage of the biosensor 1.

As shown in Fig. 7; the biosensor 1 is inserted into the insertion part 25a of the lower cover member 25. Next, the gasket 24 is mounted. As shown in Fig. 6, the positions of the biosensor 1 and the gasket 24 are adjusted so that the reference electrode layer 11, the counter electrode 13, and the temperature detection means 14, and the enzyme film 16 are exposed to the inside of the opening 24a of the gasket 24. Next, the transport pipes 22a and 22b are inserted into the transport pipe holes of the upper cover member 23. The upper cover member 23 is mounted on top of the gasket 24. In order to seal the sample chamber 21, the laminate is secured by inserting both ends into the fasteners 26a and 26b with its stacked layers being pressed together.

Fig. 8 shows one example for the use of the biosensor cell 20. The biosensor cell 20 is built into a continuous measurement device of an artificial pancreas device and the like. The transport pipe 22a of the biosensor cell 20 is connected to the collection part (not shown) which collects the sample solution, for example, a catheter inserted into the vein of a patient or a tank which dilutes and stores the blood collected from patients, or the like. The other transport pipe 22b is connected to a waste tank (not shown). Transport pipe 22b has a transport means 32, such as a pump or the like. The sample solution flows in and out of the sample chamber 21 due to the transport means 32.

The wires 11a, 12a, and 13a of the biosensor 1 are each connected to the measurement part 30 via an interconnection 30a which is connected to the wires 11a, 12a, and 13a at the end of the supporting layer 10. The wires 14a and 14b of the biosensor I are connected to the temperature control part 31 via an interconnection 31a which is connected to the wires 14a and 14b at the end of the supporting layer 10. In addition, the wires 15a and 15b of the biosensor 1 are each connected to the temperature control part 31 via an interconnection 31b which is connected to the wires 15a and 15b at the end of the supporting layer 10. The measurement part 30 has a potentiostat function for electrochemical measurement. In addition, the temperature control part 31 receives a signal from the temperature detection means 14, and based on this signal, current to the heater member 15 is adjusted in order to maintain the temperature of the sample solution and the components at a desired temperature.

In this manner, the biosensor cell 20 is built into a continuous measurement device and is used to make continuous measurement possible. The action of the continuous measurement device with the built-in biosensor cell 20 is described.

A warm-up sample solution, for example biological saline, is prepared in the collection part. By starting up the pump 32, the warm-up sample solution follows the path shown by arrows B1-B5 of Fig. 8 and flows into and out of the sample chamber 21 and is transported to a waste tank that is not shown.

When the warm-up sample solution is being transported, the measurement part 30 and the temperature control part 31 both begin operation, and the electric potential of each of the reference electrode layer 11, the working electrode layer 12, and the counter electrode 13 are measured. At the same time, the temperature detection means 14 inside the sample chamber 21 detects the temperature of the sample solution and the components. This signal is transmitted to the temperature control part. Based on the signal from the temperature detection means 14, if the temperature of the sample solution and the components is below a prescribed temperature, in the case of the artificial pancreas device this is approximately 37 degrees C, a current flows through the heater member 15 from the temperature control part 31. As a result, the heater member 15 generates heat, and the sample solution and the components inside the sample solution chamber 21 is heated via the supporting layer 10 of the biosensor 1. During this operation, the temperature detection means 14 is constantly detecting the temperature of the sample solution and the components and is transmitting the signal to the temperature control part 31. As a result, once the temperature of the sample solution and the components which is heated by the heater member 15 reaches the prescribed temperature, the current from the temperature control part 31 to the heater member 15 is stopped. Therefore, during the measurement, the temperature detection means 14 is constantly monitoring the temperature of the sample solution and the components, and this signal is transmitted to the temperature control part 31, and based on the signal transmitted from the temperature detection means 14, the temperature control part 31 determines the need for heating, and if heating is needed, a current flows to the heater member 15. Therefore, while the sample solution is being transported, the temperature of the sample solution is always maintained at a prescribed temperature.

In this manner, with the biosensor cell 20, the sample solution and the components inside the sample chamber 21 is heated and the temperature is adjusted to a prescribed temperature by the temperature detection means 14 and the heater member 15 which are formed on the surface of the supporting layer 10 of the biosensor 1. As a result, temperature adjustment is easy. Therefore, the temperature of the sample solution and the components is adjusted to the desired measurement temperature in a short period of time. In addition, there is a rapid response to temperature changes in the sample solution. The temperature of the sample solution and the components is always maintained at a constant temperature. Therefore; there is temperature stability in the biosensor cell 20, and the measurement of the biological materials and the like is highly precise.

Next, a glucose measurement mechanism by this continuous measurement device will be described. In order to measure the glucose concentration in a sample solution, the sample solution is transported from the collection part and introduced into the sample chamber 21. The enzyme film 16 is formed on top of the working electrode layer 12 and is exposed to the sample chamber 21. Due to the catalytic action of the β-D-glucose oxidase which is immobilized in the enzyme film 16, glucolactone and hydrogen peroxide are generated from the glucose in the sample solution. In this regard, when the protective layer 17 coats the surface of the enzyme film 16, the glucose in the sample solution reaches the enzyme film 16 through pores of the protective film 17, and glucolactone and hydrogen peroxide are generated by the catalytic action of the glucose. The generated hydrogen peroxide is decomposed into water and oxygen by the working electrode layer 12. As a result, a current runs between the working electrode layer 12 and the reference electrode layer 11 and/or the counter electrode layer 13. This current is in proportion to the glucose concentration. The glucose concentration in the sample solution is calculated indirectly by measuring the current value.

The transport pipes 22a and 22b of the biosensor cell 20 are provided on the upper cover member 23. However, for example, the transport pipes 22a and 22b may also be provided on the gasket 24. In addition, the biosensor cell 20 is secured by the fasteners 26a and 26b which are molded into caps with c-shaped cross-sections, but the biosensor cell 20 may also be secured by members which press together such as clips and the like. Fasteners do not have to be used, and instead the upper cover member 23 and the lower cover member 25 may be provided with latches, and the biosensor cell 20 may be secured by engaging these latches. Furthermore, the biosensor cell 20 is formed as an approximately rectangular shape in which the plate-form upper cover member 23, and the sheet-form gasket 24, and the plate-form lower cover member 25 are layered. However, the shapes of the upper cover member 23, the gasket 24, and the lower cover member 25 may be altered to other shapes, for example cylindrical shapes.

Fig. 8 shows one usage example of the biosensor cell 20. The biosensor cell 20 is built into a continuous measurement device such as an artificial pancreas device or the like. However, the biosensor cell 20 may also be built into a batch type measurement device such as a simple measurement device or portable measurement device. In addition, these continuous measurement device, batch measurement device and the like are not limited to an artificial pancreas device.

Although preferred embodiments of the present invention are described above, the present invention is not limited to the above described embodiments. Other preferred embodiments of the present invention are described below.

In the biosensor of the present invention, the supporting layer may be a film or a plate. The supporting layer may be flexible or rigid. When the supporting layer is a film with a thickness of, for example, 100-250 µm, the electrode layers formed on one side of the surface of the supporting layer, and the heater member formed on the other side of the surface can be provided close to each other, thereby making temperature measurement of the side of the electrodes more accurate.

Further, in the supporting layer, as shown in Fig. 9, a heater member 41, a supporting layer 42 and an electrode layer 43 can be stacked in that order on a surface of a substrate 40 formed from a material having rigidity. Here, the supporting layer 42 can be formed from an insulating material as a resist layer by photo-etching technology.

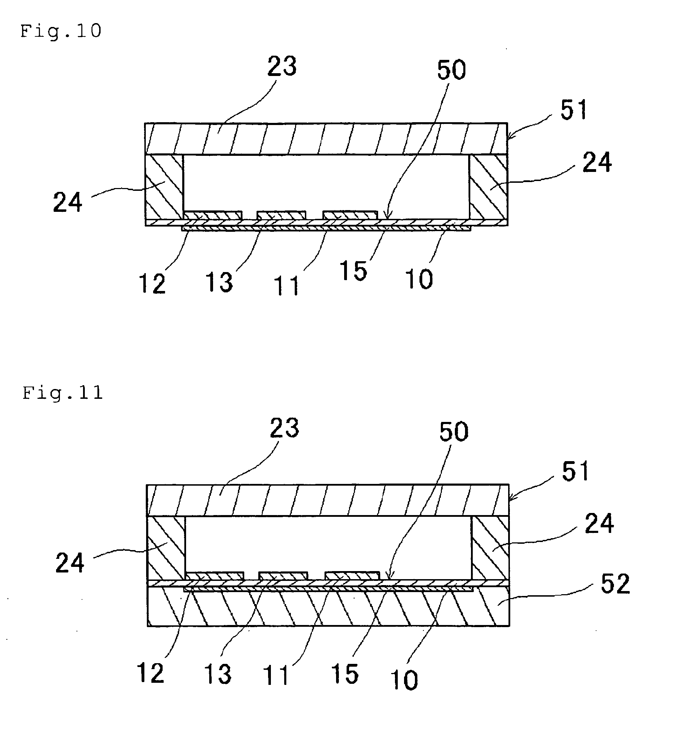

When the supporting layer is a film having rigidity, as shown in Fig. 10, a biosensor 51 can be formed by assembling one onto another the upper cover member 23, the gasket 24 and a biosensor 50, and joining them. When the supporting layer has flexibility like a film, which does not allow the supporting layer to keep its own shape, the biosensor 51 can be formed by assembling one onto another the upper cover member 23, the gasket 24, the biosensor 50 and a lower member 52, and joining them, as shown in Fig. 11.

### EMBODIMENTS

The following is an embodiment of the present invention. The present invention is not limited by these experimental examples.

### (Embodiment 1)

Polyethylene was molded into a plate of 40 mm in length, 8.5 mm in width, and 0.5 mm in thickness to form the supporting layer 10. Next, in the pattern shown in Fig. 1, the wires 11a, 12a, 13a, 14a, 14b, 15a, and 15b of 10 µm in thickness, and ground layers 11b, 12b, and 13b of 10 µm in thickness were screen printed onto one surface of the supporting layer 10 using a silver paste with a polyester resin as a base. The width of each wire was 250 µm. The ground layer I 1b had a length of 2.5 mm, a width of 2.5 mm, and a thickness of 10 µm. The ground layer 12b had a length of 0.7 mm, a width of 0.7 mm, and a thickness of 10 µm. The ground layer 13b had a length of 9.5 mm, a width of 2.5 mm, and a thickness of 10 µm.

Next, using a carbon paste with a mixture of a polyurethane resin and vinyl chloride resin as a base, the working electrode layer 12 and the counter electrode layer 13 were formed on top of the ground layers 12b and 13b by screen printing, and the heater member 15 was also formed by screen printing. Furthermore, using a silver/silver chloride paste, the reference electrode layer 11 was formed on top of the ground layer 11b by screen printing. The reference electrode layer 11 had a length of 3 mm, a width of 3.25 mm, and a thickness of 14 µm. The working electrode layer 12 had a length of 1.2 mm, a width of 1.2 mm, and a thickness of 20 µm. The counter electrode 13 had a length of 10 mm, a width of 3.25 mm, and a thickness of 20 µm. The heater member 15 had a length of 28 mm, a width of 5 mm, and a thickness of 20 µm.

Next, a commercially available thermistor (manufactured by Ishizuka Denki Corp. Ltd., model number 364 FT), as the temperature detection means 14, was glued onto the top of the supporting layer 10 with a conductive adhesive.

Next, a mixed solution, which is obtained by uniformly mixing 4% by weight glucose oxidase (Amano Enzyme Corp. Ltd.) and PVA-SbQ (Toyo Gosei Kogyo Corp. Ltd. Product number SPP-H-13), was cast over the surfaces of the reference electrode layer 11, the working electrode layer 12 and the counter electrode layer 13. Afterwards, the coating film was dried and exposed to a light of a wavelength of 300-370 nm. An enzyme film of 20 µm in thickness was obtained. In this regard, Fig. 1 shows that the enzyme film 16 coats the reference electrode layer 11, the working electrode layer 12, the counter electrode layer 13, the temperature detection means 14 and the wires 11a, 12a, 13a, 14a, 14b, 15a and 15b. Though it is sufficient for the present invention if the enzyme film coats at least the surface of the working electrode layer, the enzyme 16 in this embodiment coats, as described above, the surfaces of the reference electrode layer 11, the working electrode layer 12 and the counter electrode layer 13 so as to form the enzyme film easily. Then, the protective film 17 that sufficiently covers the enzyme film 16 is formed by superposing a commercially available porous polycarbonate film (Osmonic Corp. Ltd. Pore size 0.2 µm) having a desired size to coat at least the surface of the enzyme film 16 that coats the reference electrode layer 11, the surface of the enzyme film 16 that coats the working electrode layer 12 and the surface of the enzyme film 16 that coats the counter electrode layer 13, and by rolling it with a roller mill. Here, the temperature detection means 14 is exposed without being coated by the protective film 17.

Next, the biosensor cell 20 containing the biosensor 1 manufactured in this way was manufactured.

First, using polycarbonate, the upper cover member 23 and the lower cover member 25 with a length of 36 mm, a width of 17 mm, and a thickness of 8 mm were molded by injection molding. In the upper cover member 23, transport pipe holes were created. In the lower cover member 25, the insertion part 25a for inserting the biosensor I was formed. In addition, the transport pipes 22a and 22b were prepared. Furthermore, using silicone rubber, the gasket 24 of 29 mm in length, 6 mm in width, and 1 mm in thickness was formed. An opening of a size of 27 mm x 4 mm was provided. The fasteners 26a and 26b were molded using stainless steel. A slit 26c was formed in the fastener 26b.

Next, the biosensor 1 was inserted in the insertion part 25a of the lower cover member 25. The gasket 24 was mounted so that the reference electrode layer 11, the counter electrode layer 13, the temperature detection means 14, and the enzyme film 16 of the biosensor 1 are exposed inside the opening 24a of the gasket 24. Furthermore, the transport pipes 22a and 22b are inserted into the transport pipe holes of the upper cover member 23. The upper cover member 23 is mounted onto the gasket 24. The fasteners 26a and 26b are placed at both ends so that the layers are pressed together to seal the sample chamber 21.

### Temperature Stability Test

The biosensor of Embodiment 1 was built into the artificial pancreas device shown in Fig. 8. The temperature stabilization time which is the time at which the measurement temperature stabilizes was measured. Evaluation was conducted by repeating the test 10 times and taking the average temperature stabilization time. The results showed that the average temperature stabilization time was very short at approximately 5 minutes.

## Claims

1. A biosensor comprising:
a supporting layer;
a reference electrode layer, a working electrode layer, a counter electrode layer, and a temperature detection means, which are formed on a front surface of the supporting layer;
an enzyme film that coats a surface of the working electrode layer; and
a heater member formed on a back surface of the supporting layer.

2. The biosensor according to claim 1, wherein the supporting layer is a film.

3. The biosensor according to claim 1, wherein the heater member is formed on a surface of a base substrate, and the supporting layer, which is formed on a surface of the heater member, is a resist insulating layer.

4. A biosensor cell comprising:
a sample chamber to which a sample solution flows in and out; and
a biosensor according to any one of claims 1-3, wherein the reference electrode layer and the counter electrode layer are positioned at the sample chamber so as to be capable of making contact with the sample solution, the temperature detection means is positioned at the sample chamber so as to be capable of measuring temperature of the sample solution, and the enzyme film, which coats the working electrode layer, is positioned at the sample chamber so as to be capable of making contact with a substance to be measured in the sample solution.

## Patentansprüche

1. Biosensor, aufweisend:
eine Trägerschicht;
eine Referenzelektrodenschicht, eine Arbeitselektrodenschicht, eine Gegenelektrodenschicht und eine Temperaturerfassungseinrichtung, die auf einer Vorderfläche der Trägerschicht ausgebildet sind;
einen Enzymfilm, der eine Oberfläche der Arbeitselektrodenschicht überzieht; und
ein Heizelement, das auf einer rückwärtigen Fläche der Trägerschicht ausgebildet ist.

2. Biosensor nach Anspruch 1, wobei die Trägerschicht ein Film ist.

3. Biosensor nach Anspruch 1, wobei das Heizelement auf einer Fläche eines Basissubstrats ausgebildet ist, und die Trägerschicht, die auf einer Fläche des Heizelementes ausgebildet ist, eine Resist-Isolierschicht ist.

4. Biosensorzelle, aufweisend:
eine Probenkammer, in welche eine Probenlösung hinein- und herausfließt; und
einen Biosensor nach einem der Ansprüche 1 bis 3, wobei die Referenzelektrodenschicht und die Gegenelektrodenschicht bei der Probenkammer so positioniert sind, dass sie fähig sind, mit der Probenlösung in Kontakt zu kommen, die Temperaturerfassungseinrichtung bei der Probenkammer so positioniert ist, dass sie fähig ist, eine Temperatur der Probenlösung zu messen, und der Enzymfilm, der die Arbeitselektrodenschicht überzieht, bei der Probenkammer so positioniert ist, dass er fähig ist, mit einer zu messenden Substanz in der Probenlösung in Kontakt zu kommen.

## Revendications

1. Biocapteur comprenant :
une couche de support ;
une couche d'électrode de référence, une couche d'électrode de travail, une couche de contre-électrode, et des moyens de détection de température, qui sont formés sur une surface avant de la couche de support ;
un film d'enzyme qui revêt une surface de la couche d'électrode de travail ; et
un organe chauffant formé sur une surface arrière de la couche de support.

2. Biocapteur selon la revendication 1, dans lequel la couche de support est un film.

3. Biocapteur selon la revendication 1, dans lequel l'organe chauffant est formé sur une surface d'un substrat de base, et la couche de support, qui est formée sur une surface de l'organe chauffant, est une couche isolante de réserve.

4. Cellule à biocapteur comprenant :
une chambre d'échantillon dans laquelle et de laquelle une solution d'échantillon entre et sort ; et
un biocapteur selon l'une quelconque des revendications 1 à 3, dans lequel la couche d'électrode de référence et la couche de contre-électrode sont positionnées au niveau de la chambre d'échantillon de façon à pouvoir faire contact avec la solution d'échantillon, les moyens de détection de température sont positionnés au niveau de la chambre d'échantillon de façon à pouvoir mesurer la température de la solution d'échantillon, et le film d'enzyme, qui revêt la couche d'électrode de travail, est positionné au niveau de la chambre d'échantillon de façon à pouvoir faire contact avec une substance à mesurer dans la solution d'échantillon.
